# FASCICULE DE BREVET EUROPEEN

(11) **EP 2 257 256 B1**
(45) Date de publication et mention de la délivrance du brevet: **06.11.2013**
(21) Numéro de dépôt: 09738290.7
(22) Date de dépôt: 25.03.2009
(51) Int. Cl.: A61F 13/08

(54) **ORTHÈSE DE COMPRESSION VEINEUSE ÉLASTIQUE À MISE EN PLACE FACILITÉE**
ELASTISCHE KOMPRESSIVE ORTHESE
ELASTIC VENOUS COMPRESSION STOCKING THAT IS EASIER TO PUT ON

(30) Priorité: 28.03.2008 FR 0801692
(43) Date de publication de la demande: 08.12.2010
(73) Titulaire: Innothera Topic International, 94111 Arcueil Cedex (FR)
(72) Inventeur: CROS, François, F-94200 Ivry Sur Seine (FR)
(74) Mandataire: Dupuis-Latour, Dominique
(86) Numéro de dépôt international: PCT/FR2009/000324
(87) Numéro de publication internationale: WO 2009/133256

(56) Documents cités:
- WO-A-02/19955
- WO-A-2006/134250
- FR-A- 2 755 006
- FR-A- 2 805 459
- FR-A- 2 882 172

## Description

L'invention concerne les orthèses de compression veineuse élastique (CVE), qui sont indiquées dans les diverses manifestations cliniques d'insuffisance veineuse des membres inférieurs.

Ces orthèses, anciennement connues sous la dénomination de "bas de contention" ou "chaussettes de contention", sont des dispositifs médicaux textiles produisant un effet thérapeutique par compression/contention des membres inférieurs, par opposition aux "bas de maintien" (ou encore "bas de soutien" ou "bas anti-fatigue") et aux "bas mode", qui ne sont pas des dispositifs médicaux à visée thérapeutique.

Les orthèses de CVE sont conçues pour produire un effet thérapeutique par compression du membre inférieur sur une étendue plus ou moins grande, avec une pression P₀ mesurée à la cheville allant de 10 à plus de 36 mmHg (soit 13 à 48 hPa, le mmHg étant toutefois d'usage courant comme unité de mesure de pression dans le domaine de la phlébologie et de la compression médicale). Ces orthèses sont réparties selon le référentiel français ASQUAL en quatre classes textiles, à savoir la classe I (13 à 20 hPa, soit P₀ ≈ 10 à 15 mmHg à la cheville), la classe II (20 à 27 hPa, soit P₀ ≈ 15 à 20 mmHg), la classe III (27 à 48 hPa, soit P₀ ≈ 20 à 36 mmHg) et la classe IV (> 48 hPa, soit P₀ > 36 mmHg).

Ces orthèses procurent généralement une compression dégressive vers le haut dans la partie située au-dessus de la cheville, comme cela est indiqué incidemment par le FR 2 755 006 A1 (Couzan et al.), parfois avec un profil spécifiquement adapté où le maximum de pression est décalé au-dessus de la cheville, comme décrit par le WO 2006/134250 A2 (Inno-théra Topic International).

On notera qu'il existe d'autres référentiels (par exemple RAL GZ 387 en Allemagne) ou d'autres pratiques qui répartissent les classes de compression de façon différente, sans changer pour autant les fonctionnalités de ce type de produits.

Pour permettre une compression forte des membres inférieurs, ces orthèses sont réalisées à partir d'une maille tricotée de texture plus ou moins serrée avec incorporation d'un fil de trame élastique, généralement un élasthanne guipé. Elles sont en outre étroitement dimensionnées en fonction du membre du patient, pour obtenir par élasticité le degré et le profil de pression recherchés.

Ceci entraîne notamment une difficulté de mise en place et de retrait, en particulier au niveau du cou de pied et du talon, et ce d'autant plus que la classe textile de l'orthèse est élevée.

Cette difficulté de mise en place est généralement accrue par le fait que ces orthèses sont prescrites pour le traitement d'affections veineuses qui touchent souvent des patients âgés, malhabiles, parfois handicapés sur le plan moteur, touchés par des phénomènes arthrosiques déformant les mains et les pieds, etc., c'est-à-dire dont la mobilité est en règle générale limitée.

Cette difficulté de mise en place des orthèses de compression est un obstacle connu des patients et des soignants, et il a été proposé de nombreux types d'accessoires à cet effet.

Ces accessoires comprennent généralement une structure métallique sur laquelle est préalablement enfilée l'orthèse, de manière à la distendre et faciliter ainsi l'introduction du pied et de la cheville du patient. Ils sont cependant tous plus ou moins difficiles à mettre en oeuvre, ce qui explique leur peu de succès auprès des patients.

L'un des buts de l'invention est de proposer une orthèse compressive qui pallie ces difficultés et facilite la mise en place et le retrait par le patient, de par ses seules propriétés intrinsèques et sans recours à aucun accessoire de distension.

Le point de départ de l'invention est la constatation du fait que la difficulté se présente principalement lorsque l'orthèse est à son maximum de distension, c'est-à-dire lorsque le patient doit faire passer à celle-ci la région située entre le cou de pied et le talon (ci-après désignée "région du cou de pied", le cou de pied étant défini comme la partie supérieure antérieure située dans la région de la courbure du pied). En effet :
- pour l'orthèse, c'est au niveau de la partie de cheville que :
   - d'une part, la circonférence de l'orthèse est la plus faible (car, morphologiquement, c'est au niveau de la cheville que le membre est le plus étroit), et que
   - d'autre part, l'orthèse exerce généralement la pression la plus forte (compte tenu des exigences de dégressivité) ;
- pour le membre, c'est la région du cou de pied qui présente en section droite la circonférence la plus importante.

En d'autres termes, pour pouvoir passer la région du cou de pied lors de la mise en place de l'orthèse, il est nécessaire de distendre cette dernière très fortement dans sa partie la plus étroite et la plus compressive (partie de cheville).

L'invention propose de modifier la rhéologie de l'orthèse, c'est-à-dire sa caractéristique d'élasticité (caractéristique pression/circonférence), dans cette partie de cheville pour faciliter la mise en place.

Essentiellement, l'invention propose de modifier cette rhéologie de manière à pouvoir distendre cette partie jusqu'à une circonférence aussi grande que celle du cou de pied (bien supérieure à la circonférence de la cheville, correspondant à la configuration permanente, "au porté", de l'orthèse) sans effort excessif et sans l'aide d'aucun accessoire.

Plus précisément, l'orthèse de l'invention comporte une partie compressive s'étendant vers le haut qui, après que l'orthèse ait été mise en place sur le membre, exerce en direction circonférentielle une force de rappel élastique propre à produire une compression du membre à un niveau de pression thérapeutique. Cette partie compressive comprend une partie de cheville pouvant prendre successivement les états suivants :
- un état libre, où la partie de cheville est non élastiquement déformée,
- un état de distension nominale au passage de la région du cou de pied durant la mise en place de l'orthèse sur le membre, état dans lequel la partie de cheville, élastiquement déformée à la circonférence C₁ du cou de pied du membre, exerce en direction circonférentielle une force de rappel élastique correspondant à une pression textile nominale de mise en place P₁, et
- un état au porté, lorsque l'orthèse est en place sur le membre pour y produire une compression à un niveau de pression thérapeutique, état dans lequel la partie de cheville, élastiquement déformée à la circonférence C₀ de la cheville du membre, exerce en direction circonférentielle une force de rappel élastique produisant une pression textile nominale au porté P₀.

De façon caractéristique de l'invention, l'un au moins des paramètres parmi : la nature des fils d'âme utilisés pour lesdits fil de tricot et fil de trame, le guipage éventuel de ces fils, et le tricotage de ces fils, est choisi de manière que, pour C₁ = 1,38 C₀, la pression textile nominale de mise en place P₁ soit inférieure ou égale à 120 % de la pression textile nominale au porté P₀.

En d'autres termes, ces paramètres sont choisis pour cantonner la pression élastique produite dans l'état distendu à une valeur notablement inférieure à ce qui est rencontré avec les orthèses de CVE traditionnelles. Les circonférences de cheville C₀ et de cou de pied C₁ sont notamment celles d'une jambe-modèle telle que définie par la norme française NF G 30-102b, Annexe B, ou d'une jambe-modèle type Hohenstein selon le référentiel allemand RAL-GZ 387.

La partie de cheville peut également prendre un état de distension maximale au passage de la région du cou de pied durant la mise en place de l'orthèse sur le membre, état dans lequel la partie de cheville, élastiquement déformée à une circonférence C₂ au-delà de la circonférence C₁ du cou de pied du membre, exerce en direction circonférentielle une force de rappel élastique correspondant à une pression textile maximale de mise en place P₂,. Dans ce cas, l'un au moins des paramètres parmi : la nature des fils d'âme utilisés pour lesdits fil de tricot et fil de trame, le guipage éventuel de ces fils, et le tricotage de ces fils, est choisi de manière que, pour C₂ = 1,79 C₀, la pression textile maximale de mise en place P₂ soit inférieure ou égale à 140 % de la pression textile nominale au porté P₀.

On va maintenant décrire un exemple de mise en oeuvre du dispositif de l'invention, en référence aux dessins annexés.
La Figure 1 est une vue schématique illustrant les différentes zones concernées par le profil d'élasticité de l'orthèse de l'invention.
La Figure 2 illustre la caractéristique d'élasticité (pression/circonférence) d'une orthèse selon l'invention.

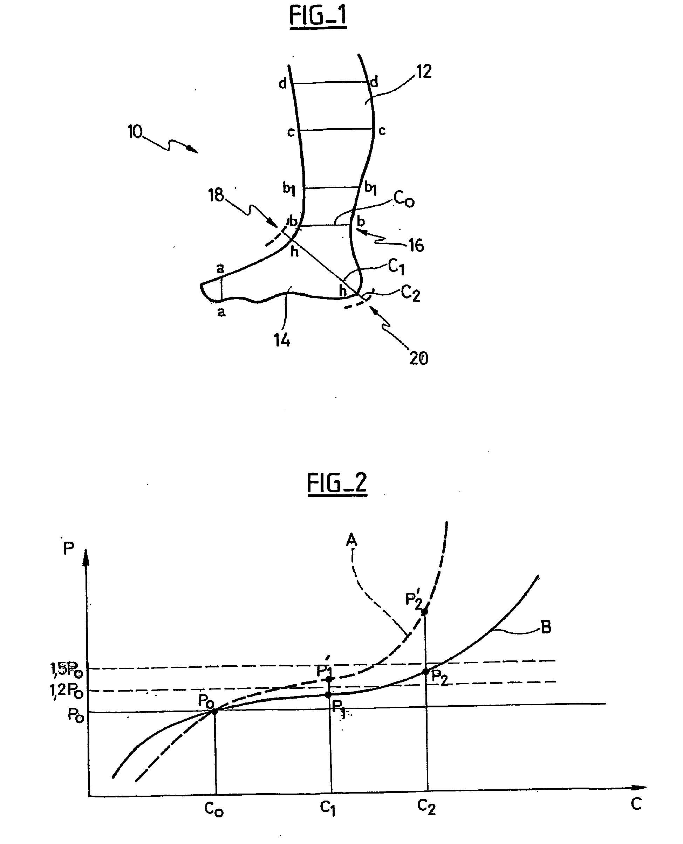

Sur la Figure 1, on a représenté les diverses parties de la partie inférieure d'une orthèse 10 qui, dans l'exemple illustré, est une orthèse fermée en partie inférieure et enveloppant le pied, par exemple une chaussette, un bas ou un collant (mais l'invention est aussi bien applicable, par exemple, à un collant à pieds ouverts).

Ces différentes parties comprennent une partie de jambe 12 s'étendant vers le haut à partir de la cheville, et une partie de pied attenante 14 s'étendant à partir de la région malléolaire jusqu'à la région des orteils. La partie de jambe 12 est une partie compressive dont la structure de maille et le dimensionnement sont choisis de manière à appliquer sur la jambe une pression thérapeutique une fois l'orthèse mise en place sur le membre.

Sous l'effet de la mise en place sur le membre, le textile tendu exerce en effet une compression résultant de la force de rappel des fibres élastiques qui composent le matériau, et l'application de ces forces de rappel élastique sur le périmètre du contour engendre en un point donné, selon la loi de Laplace, une pression locale inversement proportionnelle au rayon de courbure du contour en ce point. Cette pression est la "pression textile" telle que définie et calculée au sens de la norme française NF G 30-102b. On désignera dans la présente description par "circonférence" le périmètre dudit contour.

Le choix de la maille et des fils, ainsi que le dimensionnement des rangées de maille, sont définis de manière à appliquer des pressions prédéterminées à différentes altitudes de la jambe, par exemple à la hauteur de la cheville, au départ du mollet, au niveau du mollet, au creux poplité, etc. jusqu'en haut de cuisse dans le cas d'un bas-cuisse ou d'un collant. Ces différentes pressions sont définies pour chaque classe de contention/ compression en référence à des gabarits métrologiques tels que la jambe-modèle définie par la norme française NF G 30-102b, Annexe B, ou la jambe-modèle type Hohenstein selon le référentiel allemand RAL-GZ 387. Les diverses altitudes correspondantes sont notées conventionnellement *b* (cheville), *b1* (départ du mollet), *c* (mollet), *d* (creux poplité), ...

Le paramètre important, dans le cadre de l'invention, est la pression exercée au niveau *b* du membre, c'est-à-dire au périmètre minimal de la cheville. Dans cette région, l'orthèse exerce une pression normalisée P₀ pour une circonférence de tour de cheville C₀ de la jambe-modèle censée représenter la jambe du patient.

Lors de la mise en place ou du retrait de l'orthèse, la partie de cheville 16 de l'orthèse devra être distendue au-delà de la circonférence nominale C₀, pour permettre le passage de la région du membre située entre le cou de pied 18 et le talon 20 (ci-après "région du cou de pied"), c'est-à-dire la région du pied présentant, en section droite (section *h*-*h*), la dimension la plus importante. Le périmètre de cette section correspond à une dimension circonférentielle C₁, typiquement C₁ = 1,38.C₀ (coefficient issu du référentiel allemand RAL-GZ 387 de contrôle des textiles médicaux).

Pour prendre en compte les circonstances pratiques de la mise en place de l'orthèse, on définit également une circonférence C₂, typiquement C₂ = 1,79.C₀ correspondant à une circonférence elliptique C₁ dont le grand axe aurait été augmenté de deux largeurs de pouce, pour caractériser le passage du talon à l'aide de la main lors de la mise en place ou du retrait de l'orthèse.

Sur la Figure 2, on a illustré la caractéristique d'élasticité de l'orthèse, considérée au niveau de la partie de cheville 16 (au niveau *b-b*).

Cette caractéristique donne, en fonction de l'étirement de l'orthèse (étirement mesuré par la valeur de circonférence), la pression textile correspondante. Cette courbe traduit donc la rhéologie de l'orthèse considérée au niveau de la partie de cheville 16.

La caractéristique A indique le profil d'élasticité typique relevé pour une orthèse conventionnelle : pour la circonférence nominale C₀, la pression correspondante est la pression au porté ou pression nominale P₀, définissant notamment la classe de compression de l'orthèse. Pour une circonférence C₁ (correspondant au passage de la région du cou de pied) ou C₂ (avec une distension supplémentaire correspondant à la largeur de deux doigts), la pression exercée croît de façon très rapide, en raison des non-linéarités rhéologiques de l'orthèse, les valeurs correspondantes de la caractéristique A étant repérées P'₁ et P'₂.

L'invention propose, pour faciliter la mise en place et le retrait de l'orthèse, de modifier les propriétés rhéologiques de celle-ci dans la partie de cheville, de manière à réduire fortement la pression exercée par cette région lorsque l'orthèse est distendue, tout en conservant la même pression nominale au porté P₀.

Les difficultés de mise en place (et de retrait) sont en effet pour une plus grande part liées à l'effort qu'il faut déployer pour arriver à faire passer au niveau du cou de pied la partie tricotée correspondant à la zone de la cheville de l'orthèse. Cette difficulté est d'autant plus marquée que la pression à la cheville est importante, c'est-à-dire pour les classes textiles de pressions les plus élevées.

Plus précisément, il convient que :
- pour la circonférence C₁, la pression textile correspondante P₁ soit typiquement inférieure ou égale à 120 % de la pression nominale au porté P₀ (c'est-à-dire, en d'autres termes, que P₁ ≤ 1,2.P₀) et que
- pour la circonférence C₂ correspondant à la distension maximale prévisible, la pression textile exercée soit typiquement inférieure ou égale à 140 % de la pression nominale au porté P₀ (c'est-à-dire, en d'autres termes, que P₂ ≤ 1,4.P₀).

On a représenté en B sur la Figure 2 un profil d'élasticité correspondant relevé pour une orthèse selon l'invention, avec des exemples de valeurs P₁ et P₂ conformes aux critères indiqués ci-dessus (P'₁ et P'₂ étant les valeurs correspondantes, ne répondant pas aux critères, pour une orthèse conventionnelle).

On notera que la pression nominale au porté P₀ est identique pour l'orthèse de l'invention et pour l'orthèse conventionnelle, car il s'agit là d'une caractéristique imposée par la norme pour que l'orthèse procure précisément l'effet thérapeutique recherché, en fonction de la classe textile de compression/contention choisie.

On a ainsi réalisé des orthèses de CVE présentant les caractéristiques suivantes, pour des articles où P₀ > 20 mmHg (classes III et IV françaises), c'est-à-dire ceux pour lesquels le problème de la difficulté de mise en place se pose avec le plus d'acuité :
- bas réalisés à partir d'un fil de trame guipé avec des fils inélastiques : P₁/P₀=1,1 et P₂/P₀ = 1,2 ;
- chaussettes réalisées à partir d'un fil de trame guipé avec des fils inélastiques : P₁/P₀ = 1,2 et P₂/P₀ = 1,4 ;
- bas réalisés à partir d'un fil de trame guipé avec des fils élastiques, ou à partir d'un fil de trame non guipé : P₁/P₀ = 1,1 et P₂/P₀ = 1,2 ;
- chaussettes réalisées à partir d'un fil de trame guipé avec des fils élastiques, ou à partir d'un fil de trame non guipé : P₁/P₀ = 1,1 et P₂/P₀ = 1,2.

Le profil d'élasticité modifié selon l'invention peut être obtenu par divers moyens : par sélection des fibres utilisées pour le fil de tricot et/ou pour le fil de trame de l'orthèse, par un guipage spécifique de ces fils, par des paramètres spécifiques de tricotage, ou encore par une combinaison de ces divers choix.

Plus précisément, si l'on considère une orthèse conventionnelle, celle-ci peut être décrite comme étant la résultante :
- d'un fil de trame T, composé d'une âme XA et de fils de couverture ZA et SA (S et Z caractérisant le sens de rotation de la couverture sur l'âme), et
- d'un fil de maille M, composé d'une âme XM et de fils de couverture ZM et SM,
- ces fils étant combinés en suivant un schéma défini par un jeu de paramètres de tricotage prédéfinis P.

Cette orthèse conventionnelle donne un profil d'élasticité au niveau de la cheville tel que celui décrit par la caractéristique A sur la Figure 2.

Pour obtenir une caractéristique d'élasticité telle que celle illustrée en B sur la Figure 2, il est possible de procéder de plusieurs manières :
a) en utilisant le même fil de trame T (âme XA et couvertures ZA et SA), et en le combinant avec un fil de maille M', différent de M, composé d'une âme XM' = XM et d'une couverture ZM' = ZM et SM' = SM avec un jeu de paramètres de guipage différent, avec le même jeu de paramètres de tricotage P ;
b) en utilisant le même fil de trame T (âme XA et couvertures ZA et SA), et en le combinant avec le même fil de maille M (âme XM et couvertures ZM et SM), mais avec un jeu de paramètres de tricotage P' différent de P ;
c) en utilisant le même fil de trame T (âme XA et couvertures ZA et SA), et en le combinant avec un fil de maille M', différent de M, composé de la même âme XM mais avec des couvertures Z'M et S'M différentes de ZM et SM, avec le même jeu de paramètres de tricotage P'.

Ces exemples ne sont pas limitatifs, et d'autres manières d'obtenir le profil recherché peuvent être envisagées, en variante ou en complément, en particulier par une modification du fil de trame.

Les divers matériaux utilisés peuvent être notamment : XA = élasthanne 570 dtex, SA = coton Ne 94/1, XM = élasthanne de 44 dtex, ZM = SM = polyamide 33dTex, X'M = élasthanne 17 dtex, Z'M = polyamide 60 dtex, S'M = coton Nm 1/186.

En ce qui concerne les paramètres de tricotage : P' = hauteur de maille augmentée de 50 % et tension de trame diminuée de 10 % ; P" = vitesse de broche doublée, étirage augmenté de 50 %

On notera que les valeurs et choix ci-dessus ne sont qu'indicatifs ; divers ajustements et adaptations peuvent être nécessaires, mais sont à la portée de l'homme du métier. Il s'agit d'obtenir et de modifier les caractéristiques rhéologiques de l'orthèse de manière que la pression de mise en place (lorsque la partie de cheville de l'orthèse est distendue pour permettre le passage du cou de pied) soit peu supérieure à la pression au porté, sans toutefois modifier cette dernière valeur qui conditionne l'effet thérapeutique recherché.

De préférence, lorsque les fils de trame ou de maille sont modifiés, cette modification s'applique à la totalité de l'orthèse et non seulement à la partie de cheville, de manière que le produit présente le même aspect visuel et tactile sur toute sa hauteur, sans discontinuité au niveau de la cheville.

## Revendications

1. Une orthèse compressive du membre inférieur en forme de bas, chaussette ou collant, obtenu par tricotage d'un fil de tricot et d'un fil de trame, cette orthèse (10) comportant une partie compressive (12) s'étendant vers le haut et apte, après que l'orthèse ait été mise en place sur le membre, à exercer en direction circonférentielle une force de rappel élastique propre à produire une compression du membre à un niveau de pression thérapeutique,
ladite partie compressive (12) comprenant une partie de cheville (16) apte à prendre successivement les états suivants :
- un état libre, où la partie de cheville est non élastiquement déformée,
- un état de distension nominale au passage de la région du cou de pied durant la mise en place de l'orthèse sur le membre,
état dans lequel la partie de cheville (16), élastiquement déformée à la circonférence C₁ du cou de pied (h) du membre, exerce en direction circonférentielle une force de rappel élastique correspondant à une pression textile nominale de mise en place P₁, et
- un état au porté, lorsque l'orthèse est en place sur le membre pour y produire une compression à un niveau de pression thérapeutique,
état dans lequel la partie de cheville (16), élastiquement déformée à la circonférence C₀ de la cheville (b) du membre, exerce en direction circonférentielle une force de rappel élastique produisant une pression textile nominale au porté P₀,
orthèse **caractérisée en ce que** l'un au moins des paramètres parmi :
· la nature des fils d'âme utilisés pour lesdits fil de tricot et fil de trame,
· le guipage éventuel de ces fils, et
· le tricotage de ces fils,
est choisi de manière que, pour un degré de distension nominale correspondant à C₁ = 1,38 C₀, ladite pression textile nominale de mise en place P₁ soit inférieure ou égale à 120 % de ladite pression textile nominale au porté P₀, lesdites pressions textiles étant des pressions définies et calculées selon NF G 30-102.

2. L'orthèse de la revendication 1, où lesdites circonférences de cheville C₀ et de cou de pied C₁ sont celles d'une jambe-modèle telle que définie par la norme française NF G 30-102b, Annexe B, ou d'une jambe-modèle type Hohenstein selon le référentiel allemand RAL-GZ 387.

3. L'orthèse de la revendication 1, dans laquelle la partie de cheville (16) peut également prendre l'état suivant :
- un état de distension maximale au passage de la région du cou de pied durant la mise en place de l'orthèse sur le membre,
état dans lequel la partie de cheville, élastiquement déformée à une circonférence C₂ au-delà de la circonférence C₁ du cou de pied (h) du membre, exerce en direction circonférentielle une force de rappel élastique correspondant à une pression textile maximale de mise en place P₂,
et dans lequel l'un au moins des paramètres parmi :
· la nature des fils d'âme utilisés pour lesdits fil de tricot et fil de trame,
· le guipage éventuel de ces fils, et
· le tricotage de ces fils,
est choisi de manière que, pour un degré de distension maximale correspondant à C₂ = 1,79 C₀, ladite pression textile maximale de mise en place P₂ soit inférieure ou égale à 140 % de ladite pression textile nominale au porté P₀, lesdites pressions textiles étant des pressions définies et calculées selon NF G 30-102.

## Patentansprüche

1. Kompressionsorthese der unteren Gliedmaße in Form eines Strumpfs, einer Socke oder einer Strumpfhose, erhalten durch Stricken eines Strickfadens und eines Schussfadens,
wobei diese Orthese (10) einen Kompressionsteil (12) aufweist, der sich nach oben erstreckt und in der Lage ist, nachdem die Orthese auf der Gliedmaße angebracht wurde, in Umfangsrichtung eine elastische Rückstellkraft auszuüben, die eine Kompression der Gliedmaße auf einem therapeutischen Druckpegel erzeugen kann,
wobei der Kompressionsteil (12) einen Knöchelteil (16) enthält, der nacheinander die folgenden Zustände annehmen kann:
- einen freien Zustand, in dem der Knöchelteil nicht elastisch verformt ist,
- einen Zustand der Nennausdehnung beim Durchgang durch den Bereich des Fußrückens während des Anbringens der Orthese auf der Gliedmaße, Zustand, in dem der am Umfang C₁ des Fußrückens (*h*) der Gliedmaße elastisch verformte Knöchelteil (16) in Umfangsrichtung eine elastische Rückstellkraft ausübt, die einem textilen Nenndruck des Anbringens P₁ entspricht, und
- einen Zustand beim Tragen, wenn die Orthese auf der Gliedmaße angebracht ist, um dort eine Kompression auf einem therapeutischen Druckpegel zu erzeugen,
Zustand, bei dem der am Umfang C₀ des Knöchels (*b*) der Gliedmaße elastisch verformte Knöchelteil (16) in Umfangsrichtung eine elastische Rückstellkraft ausübt, die einen textilen Nenndruck beim Tragen P₀ erzeugt,
wobei die Orthese **dadurch gekennzeichnet ist, dass** mindestens einer der folgenden Parameter:
• die Beschaffenheit der für den Strickfaden und den Schussfaden verwendeten Kernfäden,
• die möglich Umspinnung dieser Fäden, und
• das Stricken dieser Fäden,
so gewählt wird, dass für einen Nennausdehnungsgrad entsprechend C₁ = 1,38 C₀ der textile Nenndruck des Anbringens P₁ geringer als oder gleich 120 % des textilen Nenndrucks beim Tragen P₀ ist, wobei die textilen Drücke gemäß NF G 30-102 definierte und berechnete Drücke sind.

2. Orthese nach Anspruch 1, wobei die Knöchelumfänge C₀ und Fußrückenumfänge C₁ diejenigen eines Modellbeins, wie es durch die französische Norm NF G 30-102b, Anhang B, definiert ist, oder eines Modellbeins der Art Hohenstein gemäß der deutschen Vorgabe RAL-GZ 387 sind.

3. Orthese nach Anspruch 1, wobei der Knöchelteil (16) ebenfalls den folgenden Zustand annehmen kann:
- einen Zustand der maximalen Ausdehnung beim Durchgang durch den Bereich des Fußrückens während des Anbringens der Orthese auf der Gliedmaße,
Zustand, in dem der auf einen Umfang C₂ über den Umfang C₁ des Fußrückens (*h*) der Gliedmaße hinaus elastisch verformte Knöchelteil in Umfangsrichtung eine elastische Rückstellkraft ausübt, die einem maximalen textilen Druck des Anbringens P₂ entspricht,
und wobei mindestens einer der folgenden Parameter:
• die Beschaffenheit der für den Strickfaden und den Schussfaden verwendeten Kernfäden,
• die möglich Umspinnung dieser Fäden, und
• das Stricken dieser Fäden,
so gewählt wird, dass für einen maximalen Ausdehnungsgrad entsprechend C₂ = 1,79 C₀ der maximale textile Druck des Anbringens P₂ geringer als oder gleich 140 % des textilen Nenndrucks beim Tragen P₀ ist, wobei die textilen Drücke gemäß NF G 30-102 definierte und berechnete Drücke sind.

## Claims

1. A compression orthosis for the lower limb, in the form of stockings, socks or tights, obtained by knitting with a knit thread and a weft thread, this orthosis (10) comprising a compressive part (12) extending upwards and able, once the orthosis has been put onto the limb, to apply in a circumferential direction an elastic return force capable of compressing the limb to a therapeutic pressure level,
the said compressive part (12) comprising an ankle part (16) able to adopt in succession the following states:
- a free state in which the ankle part is not elastically deformed,
- a state of nominal distension as the instep region of the foot passes through it as the orthosis is being put onto the limb,
in which state the ankle part (16), elastically deformed to the circumference C₁ of the instep (h) of the limb, applies in a circumferential direction an elastic return force corresponding to a nominal donning textile pressure P₁, and
- a wearing state, when the orthosis is in place on the limb where it produces a compression at a therapeutic pressure level,
in which state the ankle part (16), elastically deformed to the circumference C₀ of the ankle (b) of the limb, applies in a circumferential direction an elastic return force that produces a nominal wearing textile pressure P₀,
which orthosis is **characterized in that**, of the following parameters:
• the nature of the core threads used for the said knit thread and weft thread,
• any wrappings these threads might have, and
• the knitting of these threads,
at least one is chosen so that, for a nominal degree of distension corresponding to C₁ = 1.38 C₀, the said nominal donning textile pressure P₁ is less than or equal to 120% of the said nominal wearing textile pressure P₀, the said textile pressures being pressures defined and calculated in accordance with NF G 30-102.

2. The orthosis of Claim 1, in which the said ankle circumference C₀ and instep circumference C₁ are those of a model leg as defined by French standard NF G 30-102b, annex B, or of a Hohenstein-type model leg according to German reference RAL-GZ 387.

3. The orthosis of Claim 1, in which the ankle part (16) can also adopt the following state:
- a state of maximum distension as the instep region of the foot passes through it as the orthosis is being put onto the limb,
in which state the ankle part, elastically deformed to a circumference C₂ beyond the circumference C₁ of the instep (h) of the limb, applies in a circumferential direction an elastic return force corresponding to a maximum donning textile pressure P₂,
and in which, of the following parameters:
• the nature of the core threads used for the said knit thread and weft thread,
• any wrapping these threads might have, and
• the knitting of these threads,
at least one is chosen so that, for a maximum degree of distension corresponding to C₂ = 1.79 C₀, the said maximum donning textile pressure P₂ is less than or equal to 140% of the said nominal wearing textile pressure P₀, the said textile pressures being pressures defined and calculated in accordance with NF G 30-102.
